(19) Europäisches Patentamt
European Patent Office
Office européen des brevets

(11) Numéro de publication: **0 012 641**
**B1**

# (12) FASCICULE DE BREVET EUROPEEN

(45) Date de publication du fascicule du brevet: **12.05.82**

(21) Numéro de dépôt: **79400873.0**

(22) Date de dépôt: **14.11.79**

(51) Int. Cl.³: **C 07 J 5/00,** C 07 J 7/00, C 07 J 43/00, C 07 J 51/00, C 07 J 53/00; A 61 K 31/57, A 61 K 31/58 //C07D317/72, C07J17/00

(54) **Dérivés 2-substitués du 19-nor-pregn-4-ène-3,20-dione, procédé pour leur préparation et compositions pharmaceutiques les renfermant.**

(30) Priorité: **13.12.78 FR 7835046**

(43) Date de publication de la demande:
**25.06.80 Bulletin 80/13**

(45) Mention de la délivrance du brevet:
**12.05.82 Bulletin 82/19**

(84) Etats contractants désignés:
**AT BE CH DE FR GB IT NL SE**

(56) Documents cités:
**CH - A - 473 106**
**FR - A - 1 476 572**
**FR - A - 2 322 606**
**FR - A - 2 364 655**

(73) Titulaire: **ROUSSEL-UCLAF**
**102, route de Noisy Boîte postale no.9**
**F-93230 Romainville (FR)**

(72) Inventeur: **Nedelec, Lucien**
**45, boulevard de l'Ouest**
**F-93340 Le Raincy (FR)**
Inventeur: **Torelli, Vesperto**
**5, rue de la Convention**
**F-94700 Maisons Alfort (FR)**
Inventeur: **Fournex, Robert**
**8, rue du Commandant Rivière**
**F-75008 Paris (FR)**

(74) Mandataire: **Tonnellier, Marie-José et al,**
**102, route de Noisy Boîte postale no 9**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 012 641 B1

Dérivés 2-substitués du 19-nor-pregn-4-ène-3,20-dione, procédé pour leur préparation et compositions pharmaceutiques les renfermant

La présente invention concerne de nouveaux dérivés 2,2-diméthyl 19-nor stéroïdes, leur procédé de préparation, leur application comme médicament et les compositions pharmaceutiques les renfermant.

L'invention a pour objet les composés de formule I':

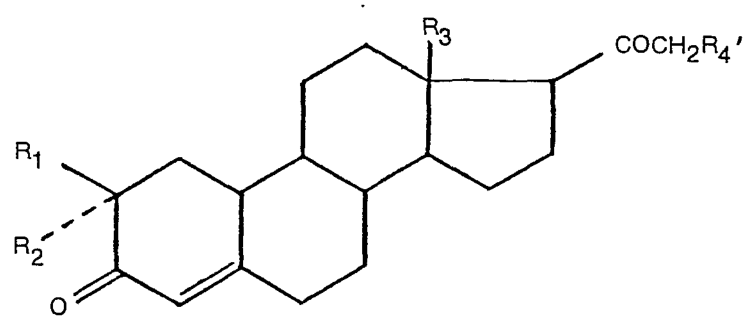

I'

dans laquelle $R_3$ représente un radical alkyle renfermant de 2 à 4 atomes de carbone, $R_4'$ représente un atome d'hydrogène, un radical hydroxyle, un radical acyloxy renfermant de 1 à 18 atomes de carbone ou un radical:

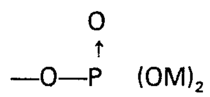

dans lequel M représente un atome d'hydrogène, un atome de sodium ou de potassium, et, ou bien $R_1$ et $R_2$, identiques ou différents, représentent un atome d'hydrogène, un radical alkyle renfermant de 1 à 4 atomes de carbone, ou un radical alkényle ou alkynyle renfermant de 2 à 6 atomes de carbone, étant entendu que seul un des substituants $R_1$ et $R_2$ peut représenter un atome d'hydrogène, ou bien $R_1$ et $R_2$ forment ensemble un radical cycloalkyle renfermant de 3 à 8 atomes de carbone.

Parmi les composés de formule I', l'invention a notamment pour objet les composés de formule I:

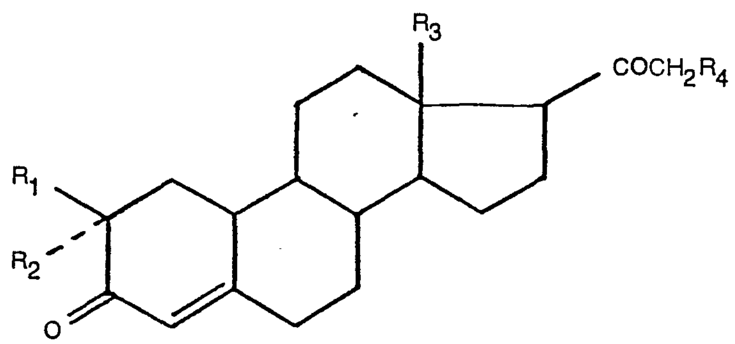

I

dans laquelle $R_4$ représente un atome d'hydrogène, un radical hydroxyle ou un radical acyloxy renfermant de 1 à 18 atomes de carbone et $R_1$, $R_2$ et $R_3$ sont définis comme ci-dessus. Lorsque $R_4'$ ou $R_4$ représente un radical acyloxy, il s'agit de préférence du reste acyloxy dérivé d'un acide aliphatique ou cycloaliphatique saturé ou insaturé et, notamment, du reste d'un acide alcanoïque tel que l'acide acétique, propionique, butyrique, isobutyrique ou undécylique, d'un acide cycloalcoylcarboxylique ou cycloalcanoïque tel que, par exemple, l'acide cyclopropyl-, cyclopentyl- ou cyclohexylcarboxylique, cyclopentyl- ou cyclohexylacétique ou propionique, de l'acide benzoïque ou métasulfobenzoïque, d'un acide phénylalcanoïque tel que l'acide phényl acétique ou phénylpropionique; il peut s'agir également d'un radical acyloxy $OCOR_4''$ dans lequel le radical $R_4''$ renferme au moins un hétéroatome choisi dans le groupe constitué par l'azote, le soufre et l'oxygène ou dans lequel $R_4''$ représente une chaîne hydrocarbonée renfermant au moins un atome d'oxygène; il peut s'agir, par exemple, du radical acyloxy $OCOR_4''$ dans lequel $R_4''$ représente un radical hétérocyclique renfermant au moins un hétéroatome comme le radical 3-pyridinyle, 4-pyridinyle, thiazolyle, 4,5-dihydrothiazolyle, oxazolyle ou imidazolyle; il peut s'agir également d'un radical acyloxy $OCOR_4''$ dans lequel $R_4''$ représente un radical $alc_1/(CH_2)_n O/_m Oalc_2$, n représentant un nombre entier pouvant varier de 1 à 8, m représentant un nombre entier pouvant varier de 1 à 6, $alc_1$ et $alc_2$, identiques ou différents, représentant un radical alcoyle linéaire ou

ramifié, saturé ou insaturé, renferment de 1 à 8 atomes de carbone, ou bien un radical $(CH_2)_pCO_2H$, p représentant un nombre entier pouvant varier de 1 à 8.

Lorsque $R_1$ ou $R_2$ représente un radical alkyle, il s'agit de préférence du radical méthyle ou éthyle.

Lorsque $R_1$ ou $R_2$ représente un radical alkényle, il s'agit de préférence du radical allyle.

Lorsque $R_1$ ou $R_2$ représente un radical alkynyle, il s'agit de préférence du radical 2-propynyle.

Lorsque $R_1$ ou $R_2$ forment ensemble un radical cycloalkyle, il s'agit de préférence du radical cyclopropyle, cyclobutyle, cyclopentyle ou cyclohexyle.

L'invention a particulièrement pour objet les composés de formule I' tels que définis précédemment, pour lesquels $R_3$ représente un radical éthyle ainsi que ceux pour lesquels $R_1$ et $R_2$ représentent chacun un radical méthyle.

L'invention a plus spécialement pour objet les composés répondant à la formule I'':

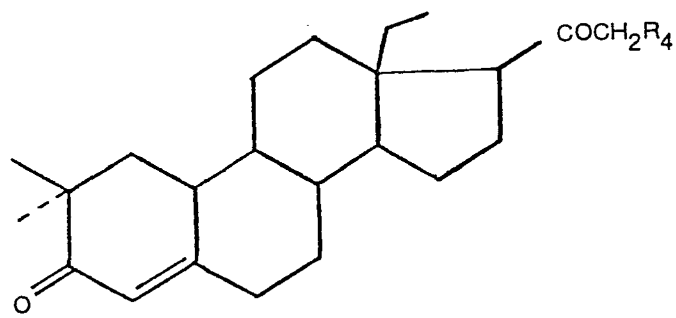

I''

dans laquelle $R_4$ conserve la même signification que précédemment.

Parmi les composés préférés de l'invention, on peut citer, notamment, les composés de formule I pour lesquels $R_4$ représente un atome d'hydrogène, ceux pour lesquels $R_4$ représente un radical hydroxy ainsi que ceux pour lesquels $R_4$ représente un radical acétoxy ou 4-pyridinyl carbonyloxy.

L'invention a tout particulièrement pour objet les composés dont la préparation est donnée plus loin dans la partie expérimentale, et, parmi ceux-ci:

— la 2,2-diméthyl 13-éthyl 21-hydroxy 18,19-dinor pregn-4-ène 3,20-dione.

Parmi les composés selon l'invention, on peut également citer:

— le sel disodique du monophosphate en 21 du 2,2-diméthyl 13-éthyl 21-hydroxy 18,19-dinor pregn 4-èn 3,20-dione,
— la 2-allyl 13-éthyl 18,19-dinor pregn-4-èn 3,20-dione,
— la 21-acétoxy 2-allyl 13-éthyl 18,19-dinor pregn-4-èn 3,20-dione,
— la 2-allyl 13-éthyl 21-hydroxy 18,19-dinor pregn-4-èn 3,20-dione,
— la 2$\alpha$ et la 2$\beta$-méthyl 13-éthyl 18,19-dinor pregn-4-èn 3,20-dione,
— la 21-acétoxy 2$\alpha$-méthyl 13-éthyl 18,19-dinor pregn-4-èn 3,20-dione,
— la 21-acétoxy 2$\beta$-méthyl 13-éthyl 18,19-dinor pregn-4-èn 3,20-dione,
— la 2$\alpha$ et la 2$\beta$-méthyl 13-éthyl 21-hydroxy 18,19-dinor pregn-4-èn 3,20-dione,
— la 2$\alpha$ et la 2$\beta$-propyl 13-éthyl 18,19-dinor pregn-4-èn 3,20-dione,
— la 21-acétoxy 2$\alpha$-propyl 13-éthyl 18,19-dinor pregn-4-èn 3,20-dione,
— la 21-acétoxy 2$\beta$-propyl 13-éthyl 18,19-dinor pregn-4-èn 3,20-dione,
— la 2$\alpha$ et la 2$\beta$-propyl 13-éthyl 21-hydroxy 18,19-dinor pregn-4-èn 3,20-dione,
— la 2,2-diéthyl 13-éthyl 18,19-dinor pregn-4-èn 3,20-dione,
— la 21-acétoxy 2,2-diéthyl 13-éthyl 18,19-dinor pregn-4-èn 3,20-dione,
— la 2,2-diéthyl 13-éthyl 21-hydroxy 18,19-dinor pregn-4-èn 3,20-dione.

Les composés de formule I' présentent d'intéressantes propriétés pharmacologiques; ils sont en particulier des antoagonistes de l'aldostérone et augmentent la diurèse hydrosodée avec conservation du potassium organique, tout en étant dénués d'effets secondaires. Il a été constaté en particulier qu'aux doses où les produits présentent une activité anti aldostérone intéressante, ceux-ci ne manifestent pas d'activité anti androgène ni d'activité anti estrogène. Ils peuvent donc être utilisés pour lutter contre l'hypertension artérielle et les insuffisances cardiaques.

L'invention a donc également pour objet les composés de formule I' à titre de médicaments. Parmi ces derniers on retient plus particulièrement le produit de l'exemple 3.

La posologie utile varie en fonction de l'affection à traiter et de la voie d'administration, elle peut aller par exemple de 10 mg à 1 g par jour chez l'adulte, par voie orale. Les composés de formule I' sont utilisés par voie buccale, rectale, transcutanée ou intraveineuse. Ils peuvent être prescrits sous forme de comprimés, de comprimés enrobés, de cachets, de capsules, de granulés, d'émulsions, de sirops, de suppositoires et de suspensions injectables.

L'invention a donc également pour objet les compositions pharmaceutiques renfermant comme principe actif au moins un composé de formule I'. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule I', caractérisé en ce que l'on condense, par réaction de WITTIG, un composé de formule II:

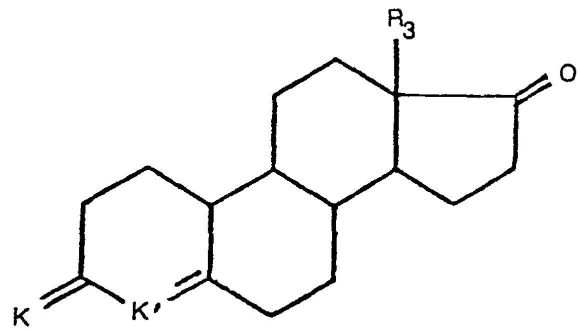

II

dans laquelle K et K' représentent un groupement cétone bloquée sous forme de cétal et $R_3$ représente un radical alkyle renfermant de 2 à 4 atomes de carbone, avec un halogénure de triphényl éthyl phosphonium de formule III:

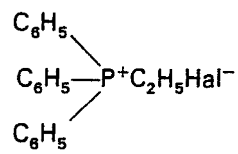

III

dans laquelle Hal représente un atome d'halogène, en présence d'une base forte, pour obtenir un composé de formule IV:

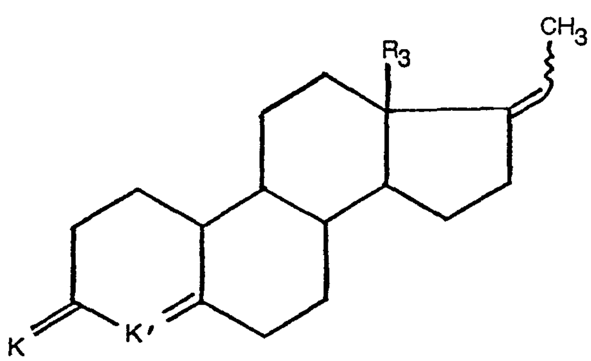

IV

sous forme d'un mélange d'isomères cis et trans, que l'on sépare, si désiré, en chacun des isomères, puis soumet soit chacun des isomères de formule IV, soit leur mélange, à l'action d'un agent d'hydroboration, puis à celle d'un agent d'oxydation en milieu alcalin et enfin à celle d'un agent de décétalisation et à celle d'un agent de cyclisation, pour obtenir un composé de formule V:

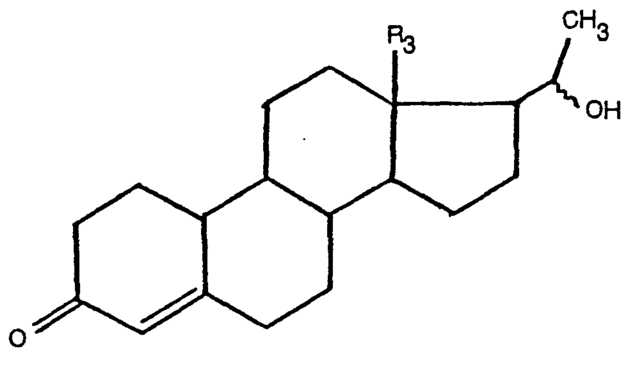

V

ramifié, saturé ou insaturé, renferment de 1 à 8 atomes de carbone, ou bien un radical $(CH_2)_pCO_2H$, p représentant un nombre entier pouvant varier de 1 à 8.

Lorsque $R_1$ ou $R_2$ représente un radical alkényle, il s'agit de préférence du radical allyle.
Lorsque $R_1$ ou $R_2$ représente un radical alkényle, il s'agit de préférence du radical allyle.

Lorsque $R_1$ ou $R_2$ représente un radical alkynyle, il s'agit de préférence du radical 2-propynyle.

Lorsque $R_1$ ou $R_2$ forment ensemble un radical cycloalkyle, il s'agit de préférence du radical cyclopropyle, cyclobutyle, cyclopentyle ou cyclohexyle.

L'invention a particulièrement pour objet les composés de formule I' tels que définis précédemment, pour lesquels $R_3$ représente un radical éthyle ainsi que ceux pour lesquels $R_1$ et $R_2$ représentent chacun un radical méthyle.

L'invention a plus spécialement pour objet les composés répondant à la formule I'':

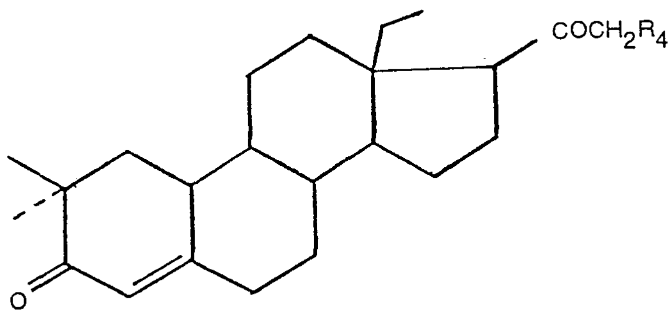

I''

dans laquelle $R_4$ conserve la même signification que précédemment.

Parmi les composés préférés de l'invention, on peut citer, notamment, les composés de formule I pour lesquels $R_4$ représente un atome d'hydrogène, ceux pour lesquels $R_4$ représente un radical hydroxy ainsi que ceux pour lesquels $R_4$ représente un radical acétoxy ou 4-pyridinyl carbonyloxy.

L'invention a tout particulièrement pour objet les composés dont la préparation est donnée plus loin dans la partie expérimentale, et, parmi ceux-ci:

— la 2,2-diméthyl 13-éthyl 21-hydroxy 18,19-dinor pregn-4-ène 3,20-dione.

Parmi les composés selon l'invention, on peut également citer:

— le sel disodique du monophosphate en 21 du 2,2-diméthyl 13-éthyl 21-hydroxy 18,19-dinor pregn 4-èn 3,20-dione,
— la 2-allyl 13-éthyl 18,19-dinor pregn-4-èn 3,20-dione,
— la 21-acétoxy 2-allyl 13-éthyl 18,19-dinor pregn-4-èn 3,20-dione,
— la 2-allyl 13-éthyl 21-hydroxy 18,19-dinor pregn-4-èn 3,20-dione,
— la 2α et la 2β-méthyl 13-éthyl 18,19-dinor pregn-4-èn 3,20-dione,
— la 21-acétoxy 2α-méthyl 13-éthyl 18,19-dinor pregn-4-èn 3,20-dione,
— la 21-acétoxy 2β-méthyl 13-éthyl 18,19-dinor pregn-4-èn 3,20-dione,
— la 2α et la 2β-méthyl 13-éthyl 21-hydroxy 18,19-dinor pregn-4-èn 3,20-dione,
— la 2α et la 2β-propyl 13-éthyl 18,19-dinor pregn-4-èn 3,20-dione,
— la 21-acétoxy 2α-propyl 13-éthyl 18,19-dinor pregn-4-èn 3,20-dione,
— la 21-acétoxy 2β-propyl 13-éthyl 18,19-dinor pregn-4-èn 3,20-dione,
— la 2α et la 2β-propyl 13-éthyl 21-hydroxy 18,19-dinor pregn-4-èn 3,20-dione,
— la 2,2-diéthyl 13-éthyl 18,19-dinor pregn-4-èn 3,20-dione,
— la 21-acétoxy 2,2-diéthyl 13-éthyl 18,19-dinor pregn-4-èn 3,20-dione,
— la 2,2-diéthyl 13-éthyl 21-hydroxy 18,19-dinor pregn-4-èn 3,20-dione.

Les composés de formule I' présentent d'intéressantes propriétés pharmacologiques; ils sont en particulier des antoagonistes de l'aldostérone et augmentent la diurèse hydrosodée avec conservation du potassium organique, tout en étant dénués d'effets secondaires. Il a été constaté en particulier qu'aux doses où les produits présentent une activité anti aldostérone intéressante, ceux-ci ne manifestent pas d'activité anti androgène ni d'activité anti estrogène. Ils peuvent donc être utilisés pour lutter contre l'hypertension artérielle et les insuffisances cardiaques.

L'invention a donc également pour objet les composés de formule I' à titre de médicaments. Parmi ces derniers on retient plus particulièrement le produit de l'exemple 3.

La posologie utile varie en fonction de l'affection à traiter et de la voie d'administration, elle peut aller par exemple de 10 mg à 1 g par jour chez l'adulte, par voie orale. Les composés de formule I' sont utilisés par voie buccale, rectale, transcutanée ou intraveineuse. Ils peuvent être prescrits sous forme de comprimés, de comprimés enrobés, de cachets, de capsules, de granulés, d'émulsions, de sirops, de suppositoires et de suspensions injectables.

L'invention a donc également pour objet les compositions pharmaceutiques renfermant comme principe actif au moins un composé de formule I'. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule I', caractérisé en ce que l'on condense, par réaction de WITTIG, un composé de formule II:

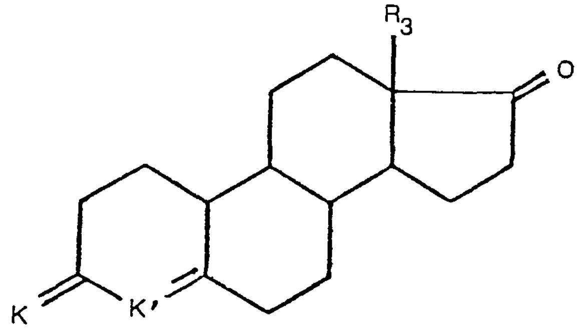

II

dans laquelle K et K' représentent un groupement cétone bloquée sous forme de cétal et $R_3$ représente un radical alkyle renfermant de 2 à 4 atomes de carbone, avec un halogénure de triphényl éthyl phosphonium de formule III:

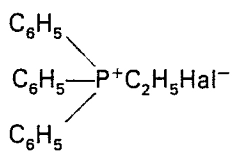

III

dans laquelle Hal représente un atome d'halogène, en présence d'une base forte, pour obtenir un composé de formule IV:

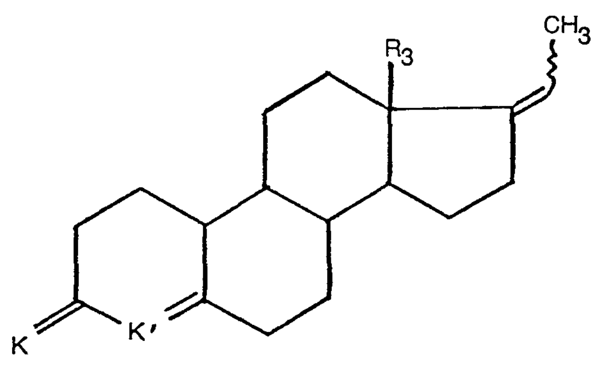

IV

sous forme d'un mélange d'isomères cis et trans, que l'on sépare, si désiré, en chacun des isomères, puis soumet soit chacun des isomères de formule IV, soit leur mélange, à l'action d'un agent d'hydroboration, puis à celle d'un agent d'oxydation en milieu alcalin et enfin à celle d'un agent de décétalisation et à celle d'un agent de cyclisation, pour obtenir un composé de formule V:

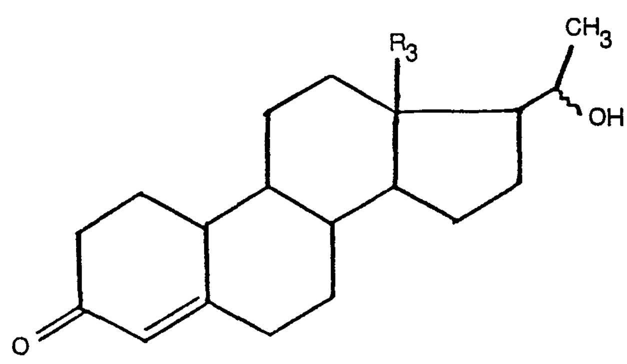

V

sous forme d'isomères 20R ou 20S ou de leur mélange, soumet soit chacun des isomères de formule V, soit leur mélange, à l'action d'un agent de blocage de la fonction alcool sous forme d'éther facilement clivable, pour obtenir un composé de formule VI:

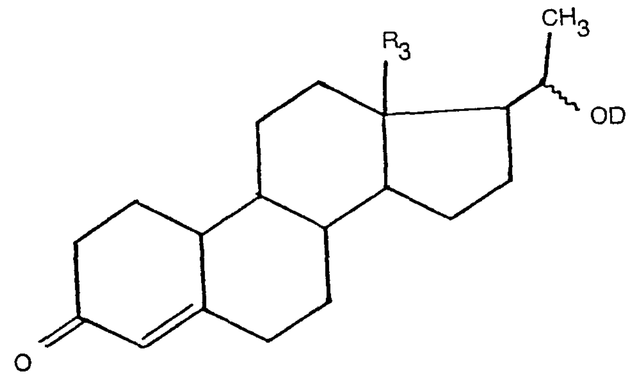

VI

sous forme d'isomères 20R ou 20S ou sous forme de leur mélange, dans laquelle OD représente un groupement éther, soumet soit chacun des isomères de formule VI, soit leur mélange, à l'action d'un halogénure d'alkyle, d'alkényle ou d'alkynyle en présence d'un agent basique à basse température, pour obtenir un composé de formule VII:

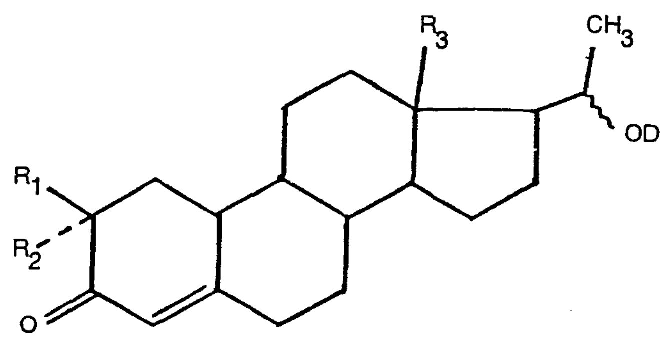

VII

dans laquelle $R_1$ et $R_2$ conservent la même signification que précédemment, sous forme d'isomère 20R ou 20S ou sous forme de leur mélange, puis soumet soit chacun des isomères de formule VII, soit leur mélange, à l'action d'un agent d'hydrolyse puis à celle d'un agent d'oxydation, pour obtenir un composé de formule $I_A$:

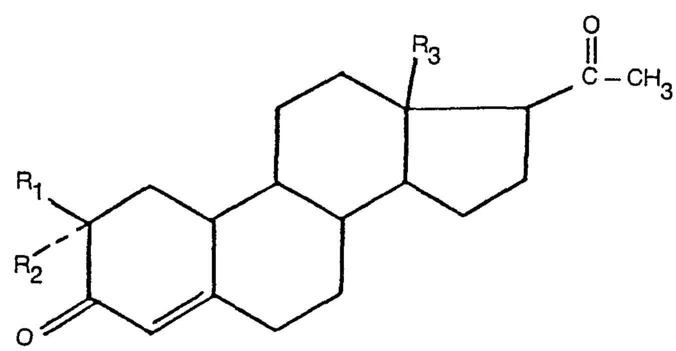

$I_A$

que l'on soumet, si désiré, ou bien à l'action du tétracétate de plomb, ou bien à l'action d'un agent d'oxalylation puis à celle d'un agent d'halogénation, pour obtenir un dérivé 21-halogéné correspondant que l'on traite par un agent d'acétoxylation, pour obtenir un composé de formule $I_B$:

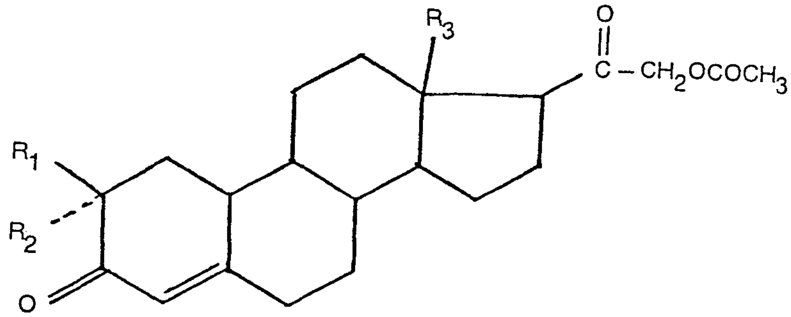

$I_B$

que l'on soumet, si désiré, à l'action d'un agent de saponification, pour obtenir un composé de formule $I_C$:

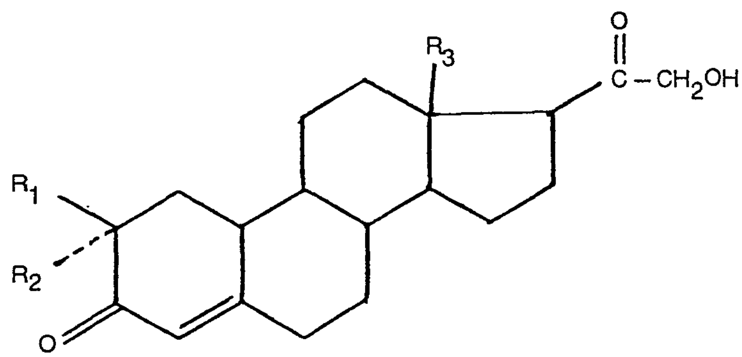

$I_C$

que l'on soumet, si désiré, soit à l'action d'un acide ou d'un dérivé fonctionnel d'acide renfermant de 1 à 18 atomes de carbone, pour obtenir un composé de formule $I_D$:

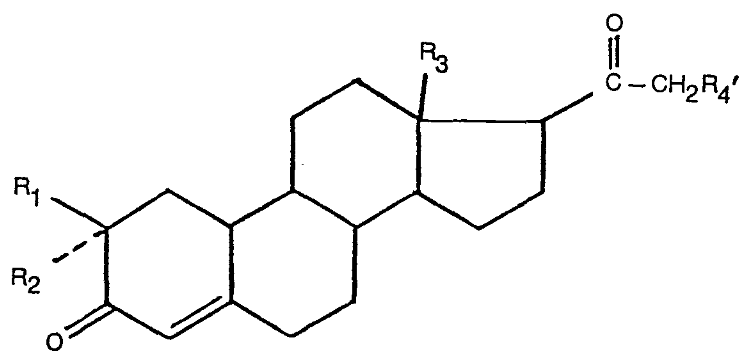

$I_D$

dans laquelle $R'_4$ représente un radical acyloxy renfermant de 1 à 18 atomes de carbone, soit à l'action d'un dérivé fonctionnel de l'acide phosphorique, pour obtenir un composé de formule $I_D$ dans laquelle $R'_4$ représente un radical:

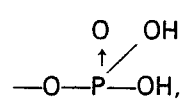

que, si désiré, l'on salifie.

Dans un mode de réalisation préférée du procédé de l'invention:

— $R_3$ représente un radical éthyle.

— K et K' représentent un groupement alkylcétal cyclique ayant de 2 à 4 atomes de carbone et, notamment, l'éthylène cétal ou le propylène cétal ou encore un dialkylcétal par exemple le diméthyl ou le diéthylcétal.

La réaction de WITTIG est réalisée dans les conditions opératoires décrites dans "Organic Reactions" *14*, 270, (1965),

— l'halogénure d'éthyltriphenylphosphonium utilisé est le bromure d'éthyltriphénylphosphonium.

— La base forte utilisée au cours de la réaction de WITTIG est un alcoolate de métal alcalin, par

exemple, le teramylate de sodium ou de potassium ou le terbutylate de sodium ou de potassium.

— La réaction de WITTIG est effectuée au sein d'un solvant tel que le benzène, le toluène, le tétrahydrofuranne, le dioxanne ou le diméthyl sulfoxyde.

— La réaction d'hydratation des oléfines de formule IV est réalisée suivant la "methode de BROWN" décrite par exemple dans "Organic Reactions" volume *13*, 1, (1963).

— L'agent d'hydroboration est le diborane, ou le diborane formé "in situ" par réaction du borohydrure de sodium sur le trifluorure de bore, ou encore un dialkylborane comme le 9-borabicyclo/3,3-1/nonane ou le diisoamylborane.

— L'agent d'oxydation en milieu alcalin utilisé est l'eau oxygénee au sein d'une solution de soude ou de potasse.

— On utilise de préférence un seul réactif comme agent de décétalisation et de cylisation, à savoir un acide fort tel que l'acide chlorhydrique ou l'acide sulfurique, mais on peut utiliser également deux agents différents. On peut par exemple utiliser un acide faible comme l'acide acétique et ensuite un agent basique comme la soude ou la potasse.

— Les isomères obtenus lors de la mise en oeuvre du procédé peuvent être séparés par les méthodes classiques de cristallisation ou de chromatographie.

— L'agent de blocage de la fonction alcool est le dihydropyran.

— $R_1$ et $R_2$ représentent chacun un radical méthyle et l'on introduit $R_1$ et $R_2$ par gem-diméthylation effectuée au moyen de l'iodure de méthyle, l'agent basique utilisé étant un alcoolate alcalin comme le terbutylate de potassium.

— La réaction de gem-diméthylation est avantageusement mise en oeuvre dans un solvant aprotique comme par exemple le tétrahydrofuranne.

— L'agent d'hydrolyse auquel on soumet le composé de formule VII est de préférence un acide comme l'acide chlorhydrique, l'acide sulfurique, l'acide acétique ou l'acide paratoluènesulfonique.

— L'agent d'oxydation auquel on soumet le composé de formule VII est le réactif d'HEILBRON-JONES, c'est-à-dire, l'anhydride chromique en solution dans l'acide sulfurique dilué.

— La réaction avec le tétracétate de plomb a lieu en présence d'éthérate de trifluorure de bore.

— L'agent d'oxalylation est l'oxalate d'éthyle.

— L'agent d'halogénation est l'iode ou le brome.

— L'agent d'acétoxylation est l'acétate de potassium.

— L'agent de saponification est de préférence une base alcaline comme la soude, la potasse, le carbonate de potassium ou carbonate acide de potassium et la réaction de saponification est réalisée de préférence au sein d'un alcool inférieur comme le méthanol ou l'éthanol.

— Le dérivé fonctionnel d'acide utilisé est de préférence un halogénure d'acide par exemple le bromure ou le chlorure ou bien un anhydride d'acide.

— La préparation du dérivé phosphoré est effectuée selon des méthodes classiques décrites dans la littérature.

La salification du dérivé phosphoré est effectuée par l'hydroxyde de sodium ou de potassium.

Les produits de formule IV, V, VI, VII obtenus lors de la mise en oeuvre du procédé décrit ci-dessus, sont des produits nouveaux et sont revendiqués dans notre demande divisionnaire n° 81200353.1.

Les produits de formule II, utilisés comme produits de départ du procédé de l'invention sont des produits connus d'une façon générale et qui peuvent être préparés selon les procédés décrits dans le brevet français 1 490 590 et dans le brevet danois 136 115.

Les exemples suivants illustrent l'invention sans toutefois la limiter.

## Exemple 1

### 2,2-diméthyl 13-éthyl 18,19-dinor pregn 4-èn 3,20-dione

*Stade A:* bis (1,2-éthanediyl)acétal cyclique de 13-éthyl 18,19-dinor 4,5-seco pregn 17(20)-èn 3,5-dione (mélange d'isomères Z et E)

On introduit 51,2 g de bromure de triphényl éthyl phosphonium dans 136 cm3 de diméthylsulfoxyde renfermant 16,8 g de terbutylate de potassium. On ajoute ensuite 27,2 g de bis (1,2-éthanediylacétal)cyclique de 13-éthyl 18,19-dinor 4,5 seco androstane 17-one. On chauffe sous agitation et sous courant d'azote à 50°C, pendant 65 heures et verse le mélange réactionnel dans 1,5 l d'eau. On extrait à l'éther. La phase orgnique est lavée à l'eau, séchée et concentrée sous pression réduite à petit volume. On sépare par filtration l'oxyde de triphénylphosphonium puis concentre le filtrat à sec.

Le résidu est purifié par chromatographie sur silice en éluant avec un mélange de cyclohexane-acétate d'éthyle (8—2). On obtient ainsi 27,35 g du produit recherché que l'on utilise tel quel dans le stade suivant.

*Stade B:* 13-éthyl 20(R,S)-hydroxy 18,19-dinor pregn 4-èn 3-one

On introduit sous atmosphère d'argon, 1,9 g d'hydroborure de sodium dans 19 cm3 de tétrahydrofuranne anhydre. On refroidit la suspension à −10°C −15°C et ajoute une solution renfermant 6,25 cm3 d'éthérate de trifluorure de bore dans 19 cm3 de tétrahydrofuranne anhydre. On agite la suspension pendant une heure à −5°C puis ajute, en 10 minutes, une solution de 10 g du produit

obtenu au stade A en solution dans 30 cm3 de tétrahydrofuranne anhydre. On amène le mélange réactionnel à température ambiante et agite pendant 1 heure 30. On refroidit ensuite le mélange réactionnel à $-10°C$ et ajoute très lentement 30 cm3 de lessive de soude puis 30 cm3 d'eau. On décante la phase aqueuse.

On lave la phase organique deux fois avec de la soude 3N et lui ajoute 10 cm3 de soude 3N. On émulsionne les deux phases par agitation et ajoute, lentement, à température ambiante 50 cm3 de perhydrol à 33%. On maintient l'agitation pendant 45 minutes puis décante la phase aqueuse et l'extrait avec de l'acétate d'éthyle. Les phases organiques jointes sont lavées avec une solution de thiosulfate de sodium à 10%, séchées et concentrées à sec sous pression réduite. On ajoute au résidu 100 cm3 d'éthanol et 50 cm3 d'acide chlorhydrique environ 5N et chauffe la solution obtenue au reflux pendant 50 minutes. On dilue ensuite à l'eau et extrait au chloroforme. On lave à l'eau la solution chloroformique, la sèche et évapore sous pression réduite. On obtient 9,8 g de produit que l'on purifie par cristallisation dans l'éther isopropylique. On obtient ainsi 5,1 g de produit recherché fondant à 140°C, qui après recristallisation dans le méthanol aqueux, fond à 142°C.

Par chromatographie de ce produit sur silice dans le mélange cyclohexane-acétate d'éthyle (1—1), on obtient l'isomère 20R pur fondant à 150°C et l'isomère 20S pur fondant à 169°C.

*Stade C:* 13-éthyl (2'RS,20RS) 20-/2'-(tétrahydropyrannyl) oxy/18,19-dinor pregn 4-èn 3-one

On dissout 13,42 g de 13-éthyl 20(R,S)hydroxy 18,19-dinor pregn 4-èn 3-one dans une solution renfermant 135 cm3 de benzène anhydre et 27 cm3 de dihydropyran. On ajoute 135 mg d'acide paratoluènesulfonique et abandonne la solution une heure à la température ambiante. On ajoute 1 cm3 de triéthylamine, lave la phase organique ä l'eau. On sèche et évapore sous pression réduite.

On obtient 19,1 g de résidu cristallisé renfermant le produit recherché qu l'on utilise tel quel dans le stade suivant.

*Stade D:* (2'RS,20RS)2,2-diméthyl 13-éthyl 20-/2'-(tétrahydropyrannyl)oxy/18,19-dinor pregn 4-èn 3-one

On dissout 6,6 g du produit obtenu au stade C dans une solution renfermant 50 cm3 de tétrahydrofuranne anhydre et 11 cm3 d'iodure de méthyle. On refroidit la solution à $-65°C$ sous atmosphère d'azote et ajoute en 20 minutes, 9,2 g de terbutylate de potassium en solution dans 45 cm3 de tétrahydrofuranne anhydre. On maintient la suspension sous agitation pendant encore 30 minutes à 65°C, puis la dilue avec de l'eau. On extrait avec du chloroforme, lave la phase organique à l'eau, la sèche et la distille à sec. On obtient 7,1 g d'extrait sec renfermant le produit recherché que l'on utilise tel quel dans le stade suivant.

*Stade E:* 2,2-diméthyl 13-éthyl 18,19-dinor pregn 4-èn 3,20-dione

On dissout les 7,1 g du produit obtenu au stade D dans une solution renfermant 70 cm3 d'éthanol et 14 cm3 d'acide chlorhydrique 2N. On chauffe à reflux pendant 1 heure puis dilue à l'eau et extrait au chlorure de méthylène. L'extrait sec (6,9 g) est dissout dans 200 cm3 d'acétone. On refroidit à 0° $-5°C$, et ajoute, en 15 minutes, 5 cm3 de réactif d'HEILBRON-JONES. On agite encore 15 minutes à 0° $-5°C$ puis détruit l'excès de réactif par addition de méthanol, dilue à l'eau et extrait au chlorure de méthylène. On obtient 5,9 g d'un produit que l'on purifie par chromatographie sur silice en éluant avec un mélange de cyclohexane-acétate d'éthyle (85—15). On obtient 2,6 g du produit recherché fondant à 179°C après recristallisation dans un mélange chlorure de methylène-éther isopropylique.

Exemple 2

21-acétoxy 2,2-diméthyl 13-éthyl 18,19-dinor pregn 4-èn 3,20-dione

a/ On dissout 1,920 g du produit obtenu à l'exemple 1 dans une solution renfermant 20 cm3 de benzène anhydre et 2 cm3 d'oxalate d'éthyle. On ajoute 450 mg d'hydrure de sodium en dispersion à 50% dans l'huile minérale et une goutte d'éthanol pour faire démarrer la réaction. On agite la suspension pendant 50 minutes jusqu'à cessation du dégagement d'hydrogène. On acidifie ensuite le milieu réactionnel avec une solution d'acide chlorhydrique sec dans l'acétate d'éthyle. On filtre les sels minéraux et on évapore le filtrat sous pression réduite. On obtient le dérivé 21-oxalylé brut, partiellement cristallié, que l'on utilise tel quel ci-après.

b/ On dissout le produit obtenu au paragraphe a/ dans une solution renfermant 24 cm3 de diméthylformamide et 4,3 cm3 de potasse méthanolique 1,3N. On ajoute 2,15 g d'acétate de potassium. On refroidit la suspension obtenue à $-5°C$ et ajoute, goutte, une solution renfermant 1,42 g d'iode dans un mélange de 3 cm3 de tétrahydrofuranne et 15 cm3 de diméthylformamide. On maintient sous agitation à $-5°C$ pendant 35 minutes après la fin de l'addition. On obtient une suspension que l'on utilise telle quelle ci-après.

c/ On ajoute 4,3 g d'acétate de potassium et chauffe la suspension sous agitation à 100°C, pendant 30 minutes. On refroidit, dilue à l'eau et extrait à acétate d'éthyle. L'extrait est purifié par chromatographie sur silice (éluant: benzène-acétate d'éthyle 9—1). On obtient ainsi 1,170 g du produit recherché fondant à 112°C après recristallisation dans l'éther isopropylique.

Exemple 3

2,2-diméthyl 13-éthyl 21-hydroxy 18,19-dinor prègn 4-èn 3,20-dione

On dissout 3,4 g du produit obtenu à l'exemple 2, dans 68 cm3 de méthanol. On chauffe la solution au reflux sous barbotage d'azote pendant 15 minutes, puis ajoute 840 mg de carbonate acide de potassium et 8,4 cm3 d'eau. On maintient au reflux sous barbotage d'azote pendant 30 minutes, refroidit et ajoute 1 cm3 d'acide acétique. On concentre sous pression réduite, dilue à leau et extrait au chlorure de méthyliène. On lave la phase organique à l'eau et la sèche. On évapore à sec. On obtient 3,06 g de produit que l'on purifie par chromatographie sur silice (éluant: benzène-acétate d'éthyle 8—2). On obtient 3 g du produit recherché fondant à 138°C après recristallisation dans l'éthanol aqueux.

Exemple 4

2,2-diméthyl 13-éthyl 21-(4-pyridinyl carbonyloxy) 18,19-dinor pregn-4-ène 3,20-dione

On met en suspension sous gaz inerte, a —10°C, 541 mg d'acide isonicotinique dans 25 cm3 de pyridine et 760 mg de chlorure de tosyle. On agite pendant 20 minutes à —10°C, ajoute 717 mg de 2,2-diméthyl 13-éthyl 21-hydroxy 18,19-dinor pregn-4-ène 3,20-dione et agite pendant une heure 30 à 10°C. On verse dans l'eau, alcalinise par addition d'une solution aqueuse de bicarbonate de sodium, extrait au chlorure de méthylène, lave la phase organique avec une solution aqueuse de bicarbonate de sodium puis à l'eau, sèche et évapore à sec. On chromatographie le résidu sur silice en éluant au mélange benzène-acétate d'éthyle (6—4) et obtient 750 mg de produit attendu qui cristallise dans l'éther isopropylique. F =156°C.

Exemple 5

Exemple de compositions pharmaceutiques

On a préparé des comprimés à 50 mg de produit de l'exemple 3, comme principe actif.

| | |
|---|---|
| Produit de l'exemple 3 | 50 mg |

Excipient (talc, amidon, stéarate de magnésium).

ETUDE PHARMACOLOGIQUE

Etude de l'activité antialdostérone chez l'animal, du produit de l'exemple 3, nommé ci-après produit A.

L'étude a été effectuée au moyen d'un test inspiré de KAGAWA C.M. (Proc. Soc. Expt. Biol. Med., *99*, 705, (1958) et de MARCUS (Endocrinology *50*, 286, (1952)).

La technique utilisée est la suivante:

Des rats mâles Sprague Dawley SPF IFFA CREDO, de 180 g sont surrénalectomisés 7 jours avant la diurèse, sous anesthésie à l'Imalgène (Kétamine) par voie intrapéritonéale à raison de 100 mg/kg. Dès l'operation et jusqu'à la veille de l'expérience, ils reçoivent comme eau de boisson du sérum physiologique.

Les animaux sont mis à jeun dix-sept heures avant la diurèse, le sérum physiologique est alors remplacé par de l'eau glucosée à 5%.

Le produit est administré par voie orale une heure avant la mise en cage.

Au moment de la mise en diurèse, les animaux reçoivent une surcharge hydrosaline par voie intrapéritonéale à raison de 5 ml par rat, de sérum physiologique à 9% et d'autre part, 1 $\mu$g/kg de monoacétate d'aldostérone en solution alcoolique à 2,5%, par voie sous-cutanée.

Les rats sont alors placés par deux en cage à diurèse, sans nourriture ni boisson, pendant quatre heures.

Au bout de ce temps, on effectue une miction forcée par pression sur la vessie et mesure le volume de l'urine recueillie.

Après rinçage soigneux des cages et de la verrerie, le volume des urines est amené à 50 cm3. Sur cette solution on effectue le dosage du sodium ete du potassium urinaires par photométrie de flamme à l'autoanalyseur.

Les résultats obtenus sont exprimés en pourcentage d'inhibition de l'activité de 1 $\mu$g/kg de monoacétate d'aldostérone injecté par voie sous-cutanée sur le log du rapport:

$$\frac{\text{concentration en sodium}}{\text{concentration en potassium}}$$

, selon la méthode de KAGAWA. Endocrinology 1960, *67* p. 125—132.

Il a été trouvé que le produit A à la dose de 2 mg/kg, par voie orale manifeste une très nette activité antialdostéronique; le pourcentage d'inhibition est à cette dose de l'ordre de 60%.

**Revendications**

1. Les composés de formule I':

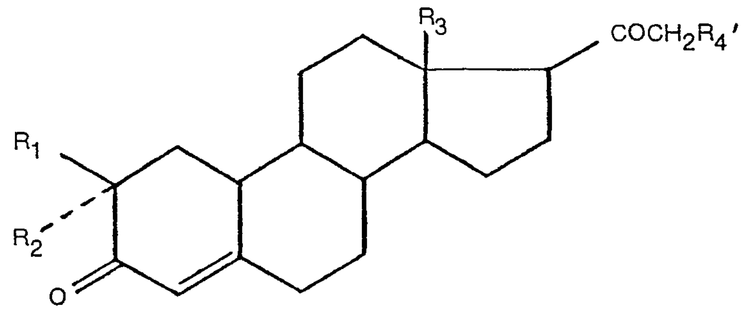

I'

dans laquelle $R_3$ représente un radical alkyle renfermant de 2 à 4 atomes de carbone, $R_4'$ représente un atome d'hydrogène, un radical hydroxyle, un radical acyloxy renfermant de 1 à 18 atomes de carbone ou un radical:

$$-O-\overset{\overset{O}{\uparrow}}{P}\ (OM)_2$$

dans lequel M représente un atome d'hydrogène, un atome de sodium ou de potassium, et, ou bien $R_1$ et $R_2$, identiques ou différents, représentent un atome d'hydrogène, un radical alkyle renfermant de 1 à 4 atomes de carbone, ou un radical alkényle ou alkynyle renfermant de 2 à 6 atomes de carbone, étant entendu que seul un des substituants $R_1$ et $R_2$ peut représenter un atome d'hydrogène, ou bien $R_1$ et $R_2$ forment ensemble un radical cycloalkyle renfermant de 3 à 8 atomes de carbone.

2. Les composés de formule I:

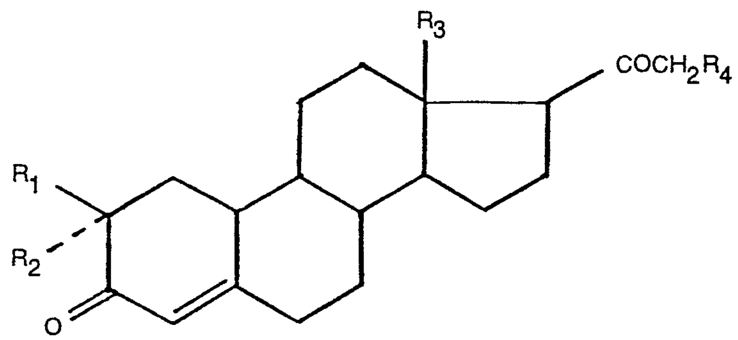

I

dans laquelle $R_4$ représente un atome d'hydrogène, un radical hydroxyle ou un radical acyloxy renfermant de 1 à 18 atomes de carbone et $R_1$, $R_2$ et $R_3$ sont définis comme à la revendication 1.

3. Les composés tels que définis à la revendication 1 ou 2, pour lesquels $R_3$ représente un radical éthyle.

4. Les composés tels que définis à la revendication 1, 2 ou 3, pour lesquels $R_1$ et $R_2$ représentent chacun un radical méthyle.

5. Les composés tels que définis à la revendication 4, répondant à la formule I'':

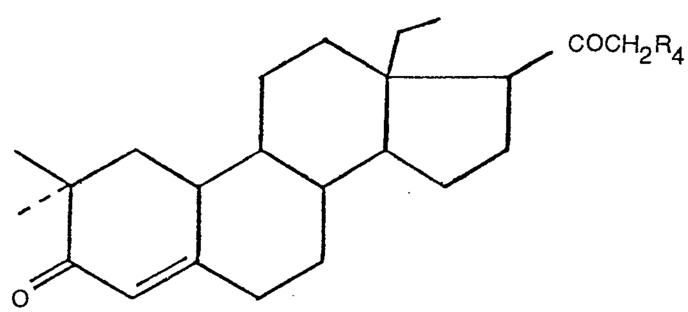

I''

dans laquelle $R_4$ conserve la même signification que dans la revendication 2.

6. Les composés de formule I, tels que définis à l'une quelconque des revendications 2 à 5, pour lesquels $R_4$ représente un atome d'hydrogène.

7. Les composés de formule I, tels que définis à l'une quelconque des revendications 2 à 5, pour lesquels $R_4$ représente un radical acétoxy.

8. Les composés de formule I, tels que définis à l'une quelconque des revendications 2 à 5, pour lesquels $R_4$ représente un radical hydroxyle.

9. Les composés de formule I, tels que définis à l'une quelconque des revendications 2 à 5, pour lesquels $R_4$ représente un radical 4-pyridinyl carbonyloxy.

10. La 2,2-diméthyl 13-éthyl 21-hydroxy 18,19-dinor pregn-4-ène 3,20-dione.

11. A titre de médicaments, les composés définis à l'une quelconque des revendications 1 à 9.

12. A titre de médicament, le composé défini à la revendication 10.

13. Les compositions pharmaceutiques renfermant, comme principe actif, au moins un médicament défini à la revendication 11 ou 12.

14. Procédé de préparation des composés de formule I tels que définis à l'une quelconque des revendications 1 à 10, caractérisé en ce que l'on condense, par réaction de WITTIG, un composé de formule II:

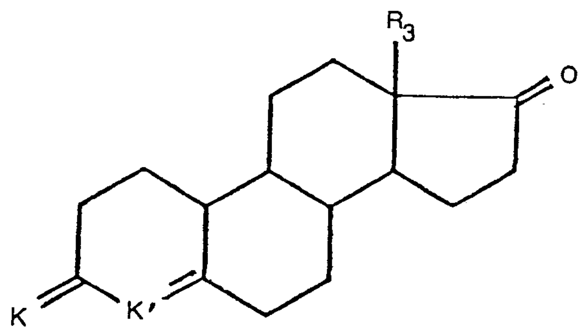

II

dans laquelle K et K' représentent un groupement cétone bloquée sous forme de cétal et $R_3$ représente un radical alkyle renfermant de 2 à 4 atomes de carbone, avec un halogénure de triphényléthyl-phosphonium de formule III:

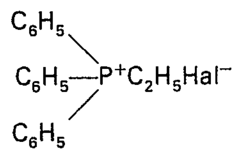

III

dans laquelle Hal représente un atome d'halogène, en présence d'une base forte, pour obtenir un composé de formule IV:

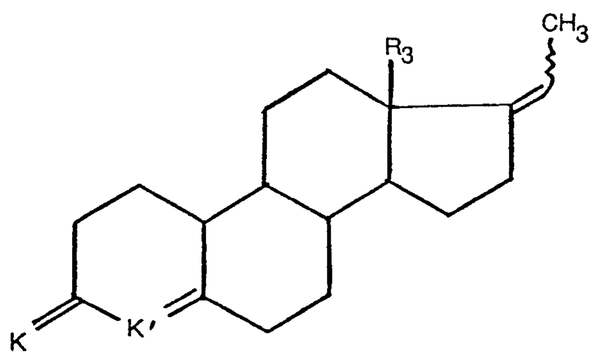

IV

sous forme d'un mélange d'isomères cis et trans, que l'on sépare, si désiré, en chacun des isomères, puis soumet soit chacun des isomères de formule IV, soit leur mélange, à l'action d'un agent d'hydroboration, puis à celle d'un agent d'oxydation en milieu alcalin et enfin à celle d'un agent de décétalisation et à celle d'un agent de cyclisation, pour obtenir un composé de formule V:

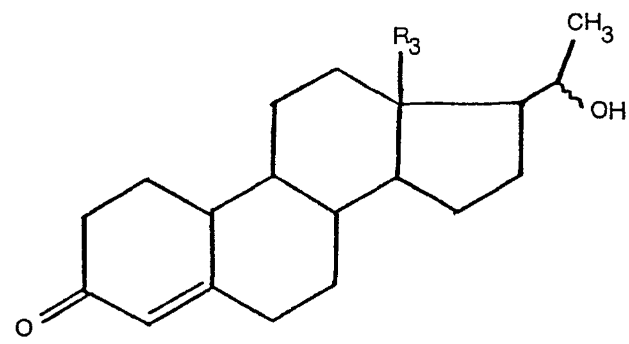

V

sous forme d'isomères 20R ou 20S ou de leur mélange, soumet soit chacun des isomères de formule V, soit leur mélange, à l'action d'un agent de blocage de la fonction alcool sous forme d'éther facilement clivable, pour obtenir un composé de formule VI:

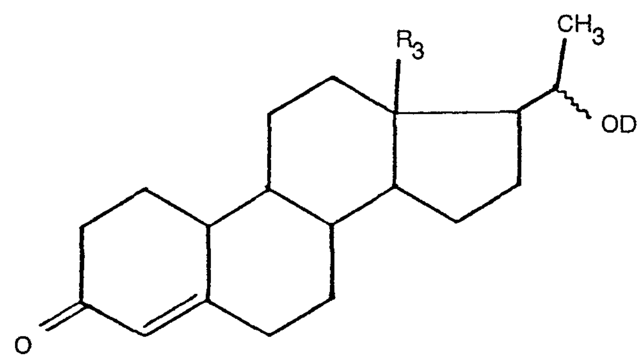

VI

sous forme d'isomères 20R ou 20S ou sous forme de leur mélange, dans laquelle OD représente un groupement éther, soumet soit chacun des isomères de formule VI, soit leur mélange, à l'action d'un halogénure d'alkyle, d'alkényle ou d'alkynyle, en présence d'un agent basique à basse température, pour obtenir un composé de formule VII:

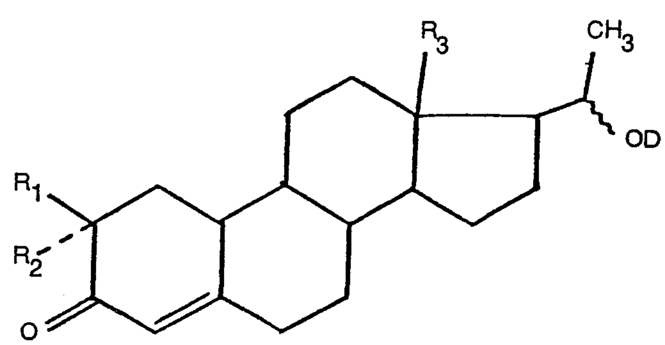

VII

dans laquelle R et $R_2$ conservant la même signification que dans la revendication 1, sous forme d'isomère 20R ou 20S ou sous forme de leur mélange, puis soumet soit chacun des isomères de formule VII, soit leur mélange, à l'action d'un agent d'hydrolyse puis à celle d'un agent d'oxydation, pour obtenir un composé de formule $I_A$:

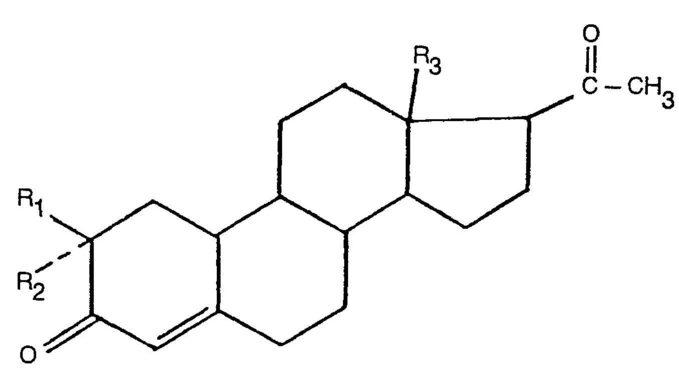

$I_A$

que l'on soumet, si désiré, ou bien à l'action du tétracétate de plomb, ou bien à l'action d'un agent d'oxalylation puis à celle d'un agent d'halogénation, pour obtenir un dérivé 21-halogéné correspondant que l'on traite par un agent d'acétoxylation, pour obtenir un composé de formule $I_B$:

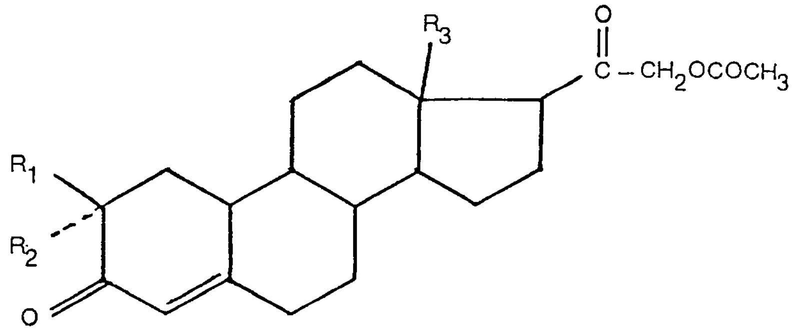

$I_B$

que l'on soumet, si désiré, à l'action d'un agent de saponification, pour obtenir un composé de formule $I_C$:

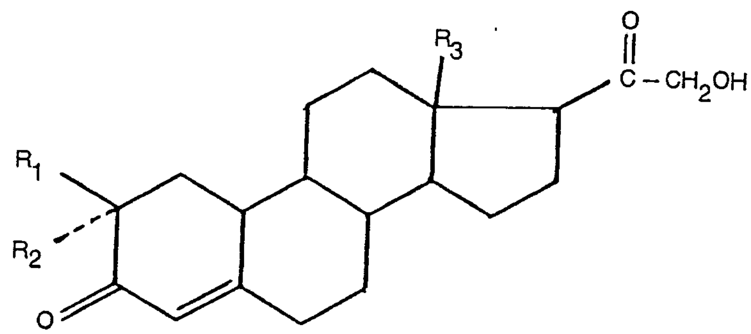

$I_C$

que l'on soumet, si désiré, soit à l'action d'un acide ou d'un dérivé fonctionnel d'acide renfermant de 1 à 18 atomes de carbone, pour obtenir un composé de formule $I_D$:

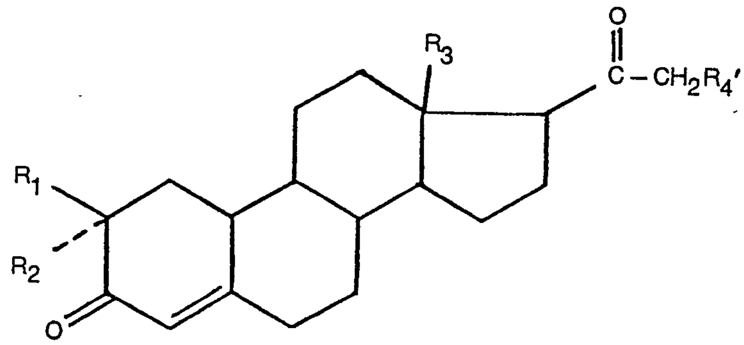

$I_D$

dans laquelle $R'_4$ représente un radical acyloxy renfermant de 1 à 18 atomes de carbone, soit à l'action d'un dérivé fonctionnel de l'acide phosphorique, pour obtenir un composé de formule $I_D$ dans laquelle $R'_4$ représente un radical:

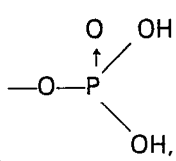

que, si désiré, l'on salifie.

## Patentansprüche

1. Verbindungen der Formel I':

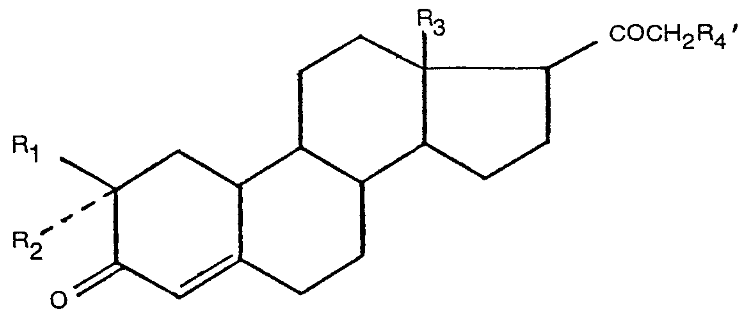

I'

worin $R_3$ einen Alkylrest mit 2 bis 4 Kohlenstoffatomen bedeutet, $R_4'$ ein Wasserstoffatom, einen Hydroxylrest, einen Acyloxyrest mit 1 bis 18 Kohlenstoffatomen oder einen Rest

$$-O-\overset{\overset{O}{\uparrow}}{P}(OM)_2,$$

worin M ein Wasserstoffatom, ein Natrium- oder Kaliumatom darstellt, bedeutet und entweder $R_1$ und $R_2$, die gleich oder verschieden sein können, ein Wassserstoffatom, einen Alkylrest mit 1 bis 4 Kohlenstoffatomen oder einen Alkenyl- oder Alkinylrest mit 2 bis 6 Kohlenstoffatomen bedeuten, unter der Voraussetzung, daß lediglich einer der Substituenten $R_1$ und $R_2$ ein Wasserstoffatom darstellen kann oder $R_1$ und $R_2$ gemeinsam einen Cycloalkylrest mit 3 bis 8 Kohlenstoffatomen bilden.

2. Verbindungen der Formel I:

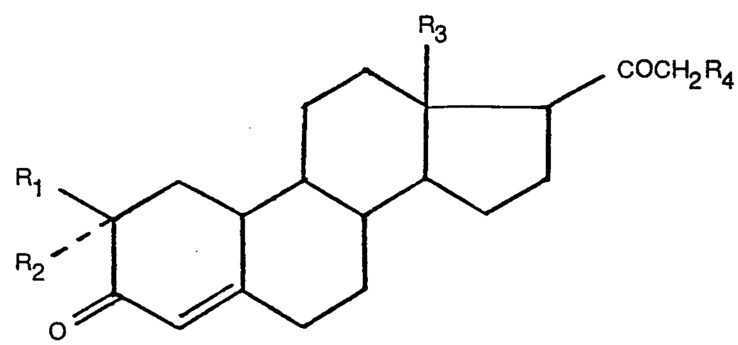

I

worin $R_4$ ein Wasserstoffatom, einen Hydroxylrest oder einen Acyloxyrest mit 1 bis 18 Kohlenstoffatomen bedeutet und $R_1$, $R_2$ und $R_3$ wie in Anspruch 1 definiert sind.

3. Verbindungen gemäß Anspruch 1 oder 2, worin $R_3$ einen Äthylrest bedeutet.

4. Verbindungen gemäß Anspruch 1, 2 oder 3, worin $R_1$ und $R_2$ jeweils einen Methylrest bedeuten.

5. Verbindungen gemäß Anspruch 4 der Formel I'':

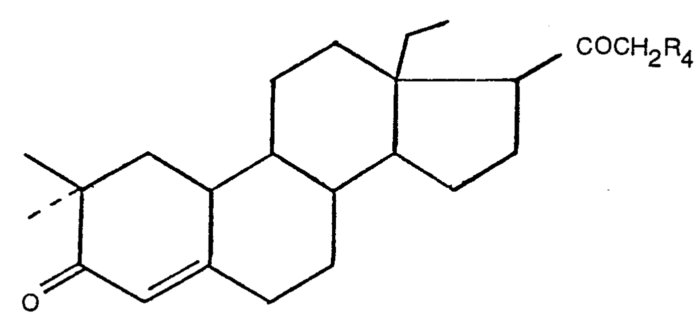

I''

worin $R_4$ die in Anspruch 2 angegebene Bedeutung besitzt.

6. Verbindungen der Formel I gemäß einem der Ansprüche 2 bis 5, worin $R_4$ ein Wasserstoffatom bedeutet.

7. Verbindungen der Formel I gemäß einem der Ansprüche 2 bis 5, worin $R_4$ einen Acetoxyrest bedeutet.

8. Verbindungen der Formel I gemäß einem der Ansprüche 2 bis 5, worin $R_4$ einen Hydroxylrest bedeutet.

9. Verbindungen der Formel I gemäß einem der Ansprüche 2 bis 5, worin $R_4$ einen 4-Pyridinylcarbonyloxyrest bedeutet.

10. 2,2-Dimethyl-13-äthyl-21-hydroxy-18,19-dinor-pregn-4-en-3,20-dion.

11. Als Arzneimittel die Verbindungen gemäß einem der Ansprüche 1 bis 9.

12. Als Arzneimittel die Verbindungen gemäß Anspruch 10.

13. Pharmazeutische Zusammensetzungen, dadurch gekennzeichnet, daß sie als Wirkstoff zumindest ein Arzneimittel gemäß Anspruch 11 oder 12 enthalten.

14. Verfahren zur Herstellung der Verbindungen der Formel I gemäß einem der Ansprüche 1 bis 10, dadurch gekennzeichnet, daß man durch Wittig-Reaktion eine Verbindung der Formel II:

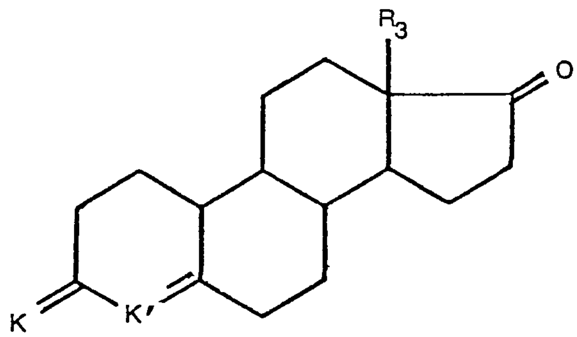

II

worin K und K′ eine in Form des Ketals blockierte Ketongruppe bedeuten und $R_3$ einen Alkylrest mit 2 bis 4 Kohlenstofffatomen darstellt, mit einem Triphenyläthylphosphoniumhalogenid der Formel III:

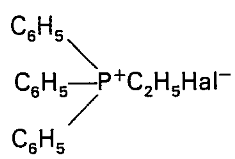

III

worin Hal ein Halogenatom bedeutet, in Gegenwart einer starken Base umsetzt, um eine Verbindung der Formel IV:

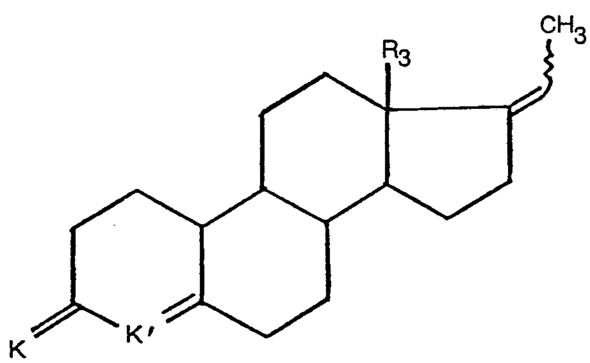

IV

in Form eines Gemisches der cis- und trans-Isomeren zu erhalten, das man gewünschtenfalls in jedes der Isomeren auftrennt, anschließend ein jedes der Isomeren der Formel IV oder deren Gemisch der Einwirkung eines Hydroborierungsmittels, anschließend derjenigen eines Oxidationsmittels in alkalischem Milieu und schließlich derjenigen eines Entketalisierungsmittels und derjenigen eines Cyclisierungsmittels unterzieht, um eine Verbindung der Formel V:

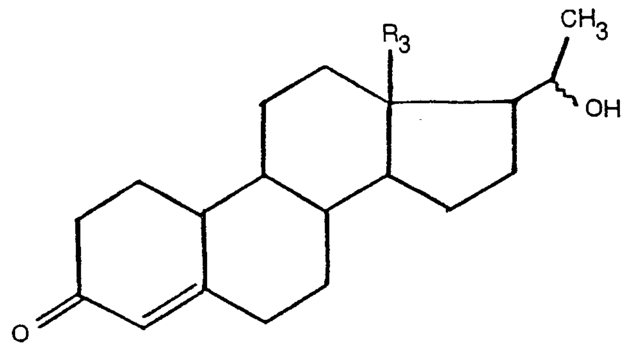

V

in Form der 20R- oder 20S-Isomeren oder in Form von deren Gemisch zu erhalten, entweder ein jedes der Isomeren der Formel V oder deren Gemisch der Einwirkung eines Mittels zur Blockierung der Alkoholfunktion in Form eines leicht spaltbaren Äthers unterzieht, um eine Verbindung der Formel VI:

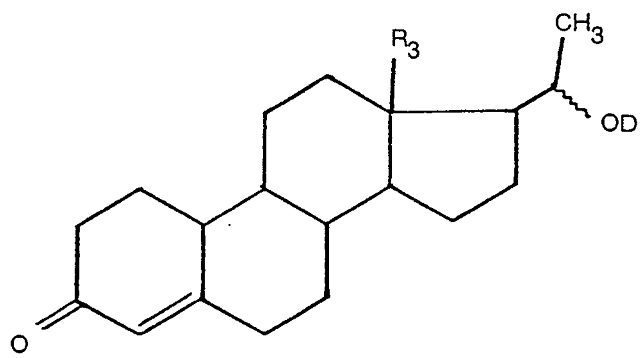

VI

in Form der 20R- oder 20S-Isomeren oder in Form von deren Gemisch, worin OD eine Äthergruppe bedeutet, zu erhalten, ein jedes der Isomeren der Formel VI oder deren Gemisch der Einwirkung eines Alkyl-, Alkenyl- oder Alkinylhalogenids in Gegenwart eines basischen Mittels bei niedriger Temperatur unterzieht, um eine Verbindung der Formel VII:

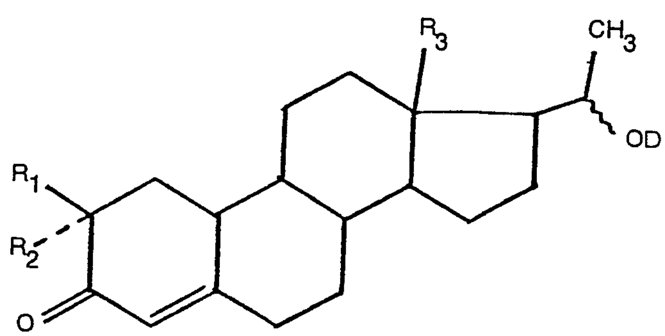

VII

worin $R_1$ und $R_2$ die in Anspruch 1 angegebene bedeutung besitzen, in Form des 20R- oder 20S-Isomeren oder in Form von deren Gemisch zu erhalten, danach ein jedes der Isomeren der Formel VII oder deren Gemisch der Einwirkung eines Hydrolysemittels und danach derjenigen eines Oxidationsmittels unterzieht, um eine Verbindung der Formel $I_A$:

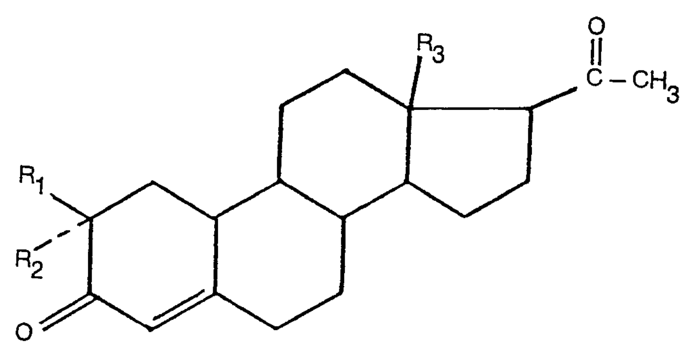

$I_A$

zu erhalten, die man gewünschtenfalls entweder der Einwirkung von Bleitetraacetat oder der Einwirkung eines Oxalylierungsmittels und danach derjenigen eines Halogenierungsmittels unterzieht, um ein entsprechendes 21-Halogen-Derivat zu erhalten, das man mit einem Acetoxylierungsmittel behandelt, um eine Verbindung der Formel $I_B$:

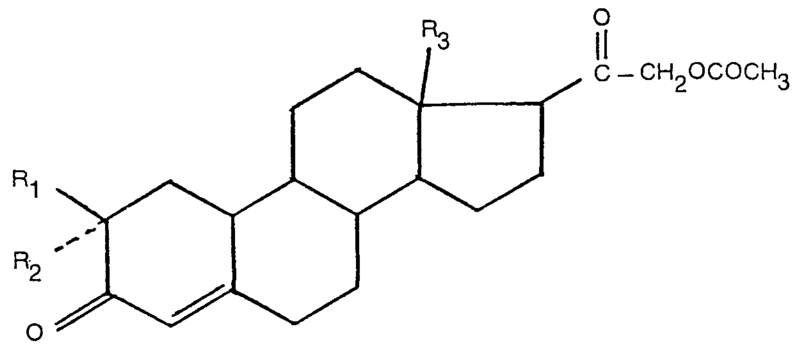

$I_B$

zu erhalten, die man gewünschtenfalls der Einwirkung eines Verseifungsmittels unterzieht, um eine Verbindung der Formel $I_C$:

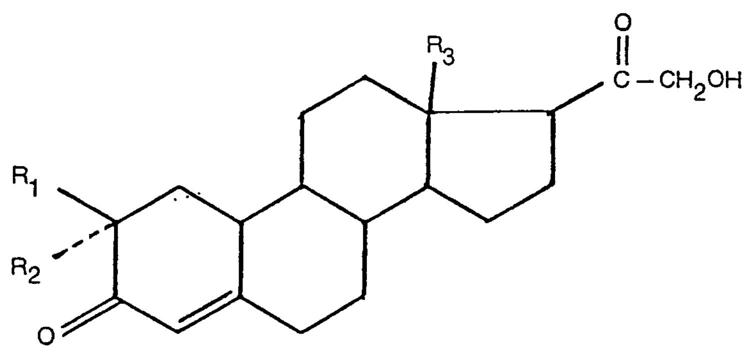

$I_C$

zu erhalten, die man gewünschtenfalls entweder der Einwirkung einer Säure oder eines funktionellen Derivats der Säure mit 1 bis 18 Kohlenstoffatomen unterzieht, um eine Verbindung der Formel $I_D$:

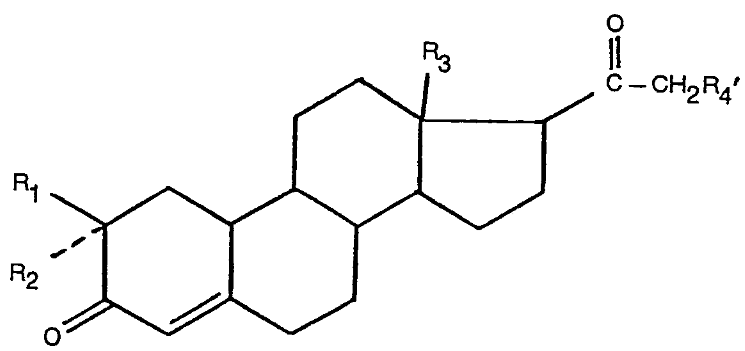

$I_D$

zu erhalten, worin $R_4'$ einen Acyloxyrest mit 1 bis 18 Kohlenstoffatomen bedeutet, oder der Einwirkung eines funktionellen Phosphorsäure-Derivats unterzieht, um eine Verbindung der Formel $I_D$ zu erhalten, worin $R_4'$ einen Rest

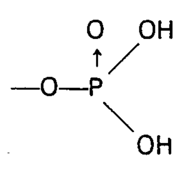

bedeutet, die man gewünschtenfalls in ein Salz überführt.

## Claims

1. Compounds of formula I':

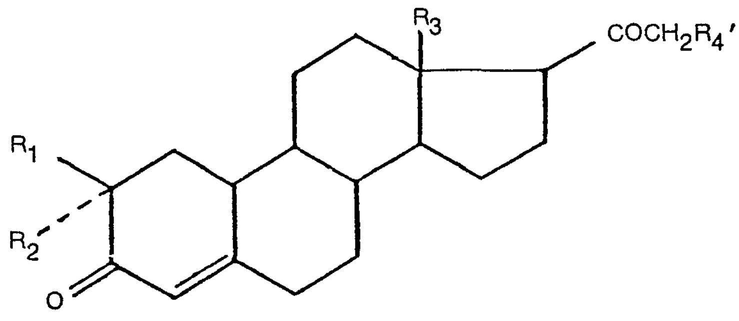

I'

in which $R_3$ represents an alkyl radical containing from 2 to 4 carbon atoms, $R_4'$ represents a hydrogen atom, a hydroxyl radical, an acyloxy radical containing from 1 to 18 carbon atoms or a radical:

$$—O—\overset{O}{\overset{\uparrow}{P}}(OM)_2,$$

in which M represents a hydrogen atom, a sodium atom or a potassium atom, and either $R_1$ and $R_2$, being the same or different, represent a hydrogen atom, an alkyl radical containing from 1 to 4 carbon atoms or an alkenyl or alkynyl radical containing from 2 to 6 carbon atoms, it being understood that only one of the substituents $R_1$ and $R_2$ can represent a hydrogen atom, or $R_1$ and $R_2$ together form a cycloalkyl radical containing from 3 to 8 carbon atoms.

2. Compounds of formula I:

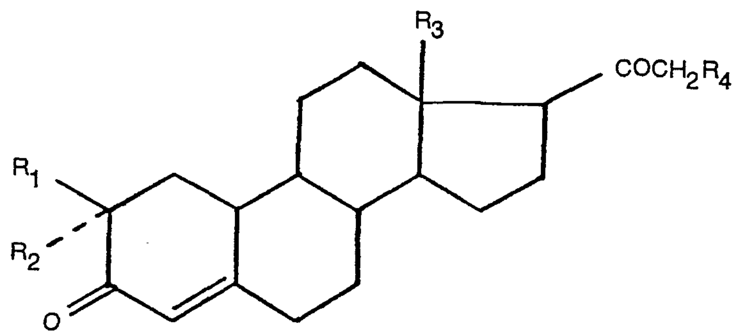

I

in which $R_4$ represents a hydrogen atom, a hydroxyl radical or an acyloxy radical containing from 1 to 18 carbon atoms and $R_1$, $R_2$, and $R_3$ are defined as in Claim 1.

3. Compounds as defined in Claim 1 or 2, for which $R_3$ represents an ethyl radical.

4. Compounds as defined in Claim 1, 2 or 3, for which $R_1$ and $R_2$ each represent a methyl radical.

5. Compounds as defined in Claim 4, corresponding to the formula I'':

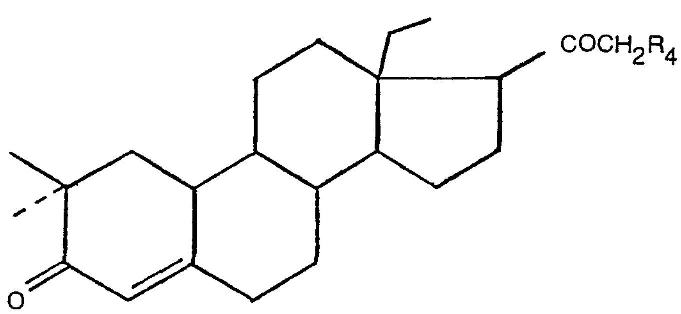

I''

in which $R_4$ keeps the same meaning as in Claim 2.

6. Compounds of formula I, as defined in any one of Claims 2 to 5, for which $R_4$ represents a hydrogen atom.

7. Compounds of formula I, as defined in any one of Claims 2 to 5, for which $R_4$ represents an acetoxy radical.

8. Compounds of formula I, as defined in any one of Claims 2 to 5, for which $R_4$ represents a hydroxyl radical.

9. Compounds of formula I, as defined in any one of Claims 2 to 5, for which $R_4$ represents a 4-pyridinyl carbonyloxy radical.

10. 2,2-dimethyl 13-ethyl 21-hydroxy 18,19-dinor pregn-4-ene 3,20-dione.

11. As medicaments, the compounds defined in any one of Claims 1 to 9.

12. As a medicament, the compound defined in Claim 10.

13. Pharmaceutical compositions containing, as active principle, at least one medicament defined in Claim 11 or 12.

14. Process for preparing the compounds of formula I as defined in any one of Claim 1 to 10, characterised in that a compound of formula II:

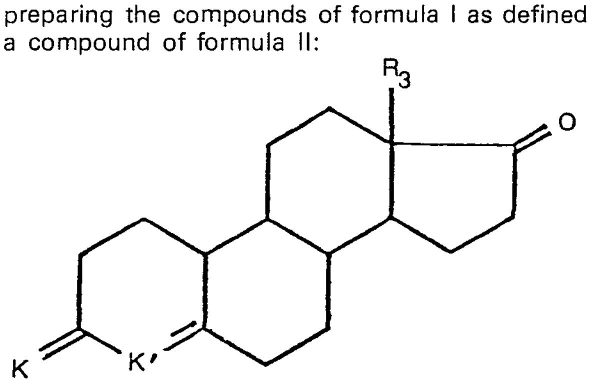

II

in which K and K' represent a blocked ketone group in the form of a ketal and $R_3$ represents an alkyl radical containing from 2 to 4 carbon atoms, is condensed by WITTIG reaction, with a triphenylethylphosphonium halide of formula III:

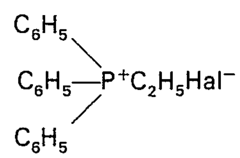

III

in which Hal represents a halogen atom, in the presence of a strong base, to obtain a compound of formula IV:

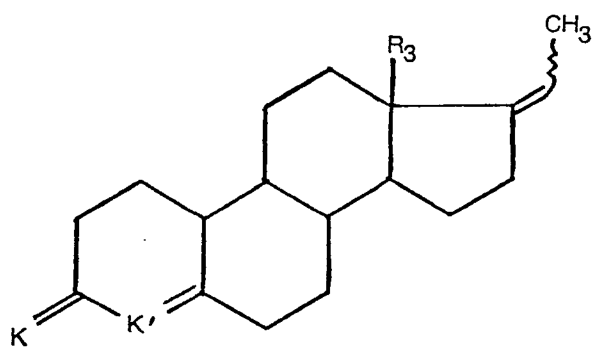

IV

in the form of a mixture of cis and trans isomers, which is separated, if desired into each of the isomers, then either each of the isomers of formula IV or their mixture is subjected to the action of a boron-hydrogenating agent, then to that of an oxidizing agent in alkaline medium and finally to that of a deketalizing agent and to that of a cyclizing agent, to obtain a compound of formula V:

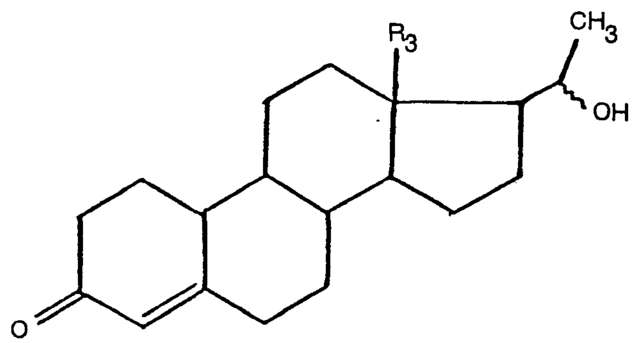

V

in the form of 20R or 20S isomers or their mixture, either each of the isomers of formula V or their mixture is subjected to the action of an agent for blocking the alcohol function in the form of an easily-cleavable ether, to obtain a compound of formula VI:

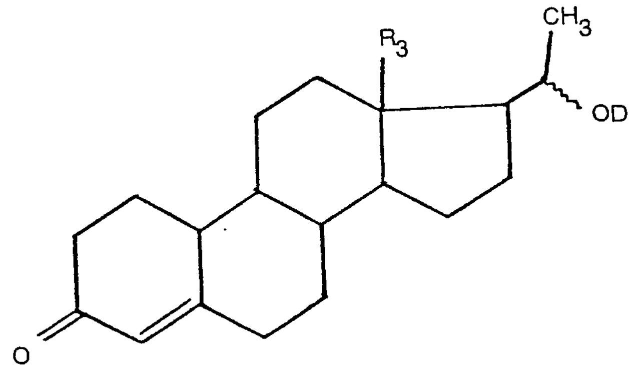

VI

in the form of 20R or 20S isomers or in the form of their mixture, in which OD represents an ether group, either each of the isomers of formula VI or their mixture is subjected to the action of an alkyl, alkenyl or alkynyl halide in the presence of a basic agent at low temperature, to obtain a compound of formula VII:

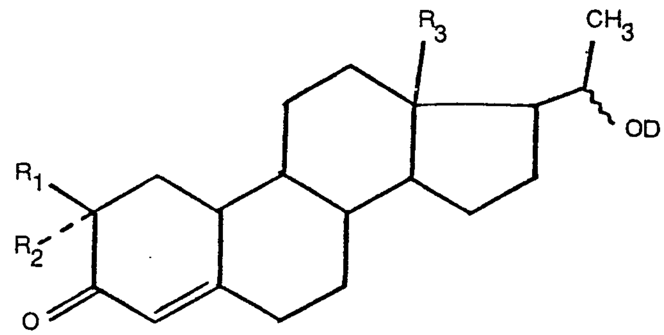

VII

in which $R_1$ and $R_2$ keep the same meaning as in Claim 1, in the form of 20R or 20S isomers or in the form of their mixture, then either each of the isomers of formula VII or their mixture is subjected to the action of a hydrolysing agent then to that of an oxidising agent, to obtain a compound of formula $I_A$:

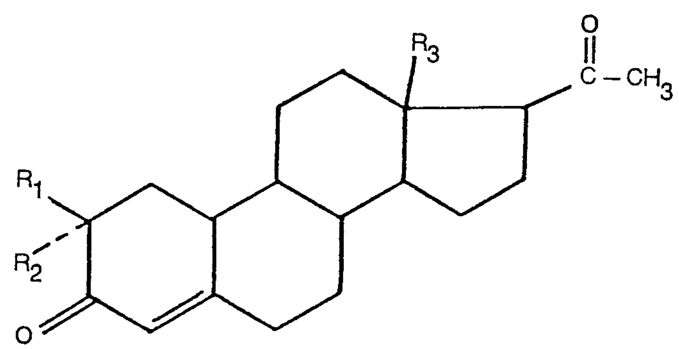

$I_A$

which is subjected, if desired, either to the action of lead tetracetate or to the action of an oxalylating agent then to that of a halogenating agent, to obtain a corresponding 21-halogenated derivative which is treated with an acetoxylating agent, to obtain a compound of formula $I_B$:

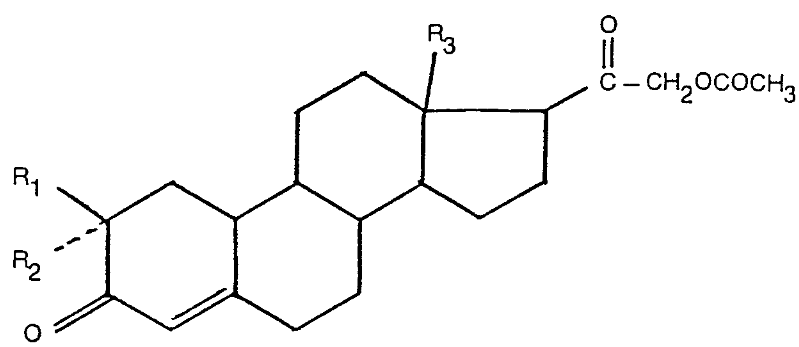

$I_B$

which is subjected, if desired, to the action of a saponifying agent, to obtain a compound of formula $I_C$:

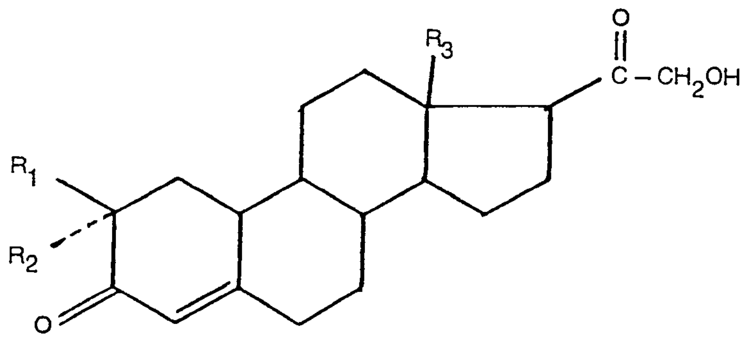

$I_C$

which is subjected, if desired, either to the action of an acid or of a functional derivative of an acid containing from 1 to 18 carbon atoms, to obtain a compound of formula $I_D$:

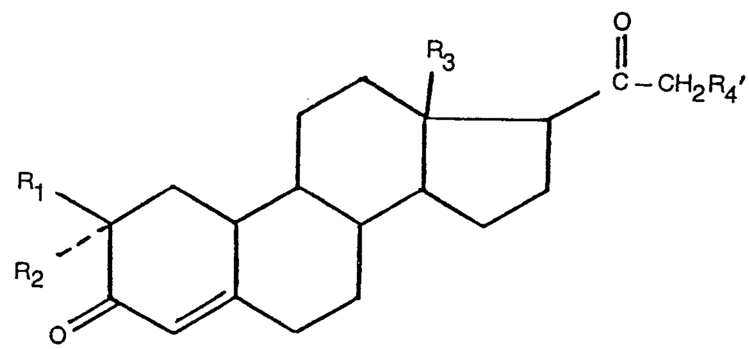

$I_D$

in which $R_4'$ represents an acyloxy radical containing from 1 to 18 carbon atoms, or to the action of a functional derivative of phosphoric acid, to obtain a compound of formula $I_D$ in which $R_4'$ represents a radical:

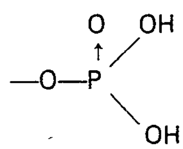

which, if desired, is salified.